(19) Europäisches Patentamt
European Patent Office
Office européen des brevets

(11) **EP 3 196 791 A1**

(12) **EUROPÄISCHE PATENTANMELDUNG**

(43) Veröffentlichungstag:
**26.07.2017 Patentblatt 2017/30**

(51) Int Cl.:
***G06F 19/00*** *(2011.01)*

(21) Anmeldenummer: **17169049.8**

(22) Anmeldetag: **02.05.2017**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**MA MD**

(71) Anmelder: **Siemens Healthcare GmbH**
**91052 Erlangen (DE)**

(72) Erfinder: **Schmidt, Sebastian**
**91085 Weisendorf (DE)**

(54) **VERFAHREN ZUR UNTERSTÜTZUNG VON MEDIZINISCHEM PERSONAL, UNTERSTÜTZUNGSSYSTEM, COMPUTERPROGRAMM UND DATENTRÄGER**

(57) Verfahren zur Unterstützung von medizinischem Personal bei der Untersuchung eines Patienten mittels wenigstens eines insbesondere eine Messung umfassenden Untersuchungsvorgangs, wobei unter Berücksichtigung von in einer ersten Datenbank (5) vorliegenden Patientendaten einer elektronischen Patientenakte, die wenigstens eine Eigenschaft des Patienten und/oder wenigstens ein Untersuchungsergebnis wenigstens eines bereits abgeschlossenen Untersuchungsvorgangs umfassen, wenigstens ein jeweils auf eine zu stellende Diagnose bezogener Prävalenzwert für eine den Patienten umfassende Personengruppe, zu der gehörig der Patient in Abhängigkeit wenigstens eines Teils der Patientendaten klassifiziert wurde, ermittelt wird und aus dem Prävalenzwert wenigstens eine auf wenigstens einen hinsichtlich der zu stellenden Diagnose durchführbaren, zukünftigen Untersuchungsvorgang bezogene Bewertungsinformation unter Berücksichtigung von in einer zweiten Datenbank (6) vorliegenden, die Sensitivität und/oder die Spezifität für den wenigstens einen zukünftigen Untersuchungsvorgang hinsichtlich der zu stellenden Diagnose beschreibenden Prädiktionsinformationen (19) erzeugt und an das medizinische Personal ausgegeben wird.

FIG 3

EP 3 196 791 A1

**Beschreibung**

Die Erfindung betrifft ein Verfahren zur Unterstützung von medizinischem Personal bei der Untersuchung eines Patienten mittels wenigstens eines insbesondere eine Messung umfassenden Untersuchungsvorgangs, ein Unterstützungssystem, ein Computerprogramm und einen elektronisch lesbaren Datenträger.

Sucht ein Patient medizinisches Personal, beispielsweise einen Arzt, auf, entschließt sich dieser in den meisten Fällen, wenigstens einen Untersuchungsvorgang anzustoßen, um das Vorliegen bestimmter Gesundheitsanomalien, insbesondere von Krankheiten, bzw. deren Ausschluss zu überprüfen, mithin eine Diagnose bezüglich einer spezifischen Gesundheitsanomalie stellen zu können. Derartige Untersuchungsvorgänge, die häufig auch als diagnostische Tests bezeichnet werden, umfassen insbesondere wenigstens eine an dem Patienten manuell und/oder mittels eines geeigneten Untersuchungsgeräts wenigstens teilweise automatisch durchzuführende Messungen, beispielsweise eine Bildaufnahme, die Detektion von Substanzen in Teilen der Anatomie und dergleichen.

US 7593913 B2 offenbart ein System zum Bereitstellen einer medizinischen Entscheidungsunterstützung für die Diagnose und die Behandlung einer Krankheit.

US 8238999 B2 offenbart ein Verfahren zum Anzeigen wenigstens eines medizinischen Befunds, bei dem zumindest ein Teil eines Körpers eines Patienten in einer Teilkörperansicht auf einem Anzeigemedium dargestellt wird.

US 8321238 B2 offenbart ein Verfahren zur Zuordnung eines Befunders zu einer Befundungsaufgabe, insbesondere einer Auswertung eines Bilddatensatzes.

US 8401872 B2 offenbart ein Verfahren zum Betreiben einer medizinischen Diagnoseeinrichtung, mit deren Hilfe medizinische Fragestellungen beantwortet werden sollen.

US 8640053 B2 offenbart ein Verfahren zur Darstellung mehrerer Bilddatensätze im Rahmen einer vergleichenden Auswertung.

Jedem diagnostischen Untersuchungsvorgang kann hierbei eine gewisse Sensitivität und Spezifität zugeordnet werden, welche in der Regel nicht 100 % betragen. Die Sensitivität gibt an, mit welcher Wahrscheinlichkeit ein tatsächlich positiver Patient als positiv erkannt wird, die Spezifität beschreibt den Anteil der negativ getesteten Personen unter allen nichtpositiven Patienten, das bedeutet, die Wahrscheinlichkeit, mit einem diagnostischen Test nicht-erkrankte korrekt zu identifizieren. Diese Werte haben in der Praxis jedoch keine sehr hohe Bedeutung, da ja gerade nicht bekannt ist, ob die Gesundheitsanomalie beim Patienten vorliegt, dieser mithin tatsächlich positiv (true positive) ist.

Als für die Praxis relevantere Werte zur Bewertung von Untersuchungsvorgängen wurden der positive prädiktive Wert und der negative prädiktive Wert (PPV bzw. NPV) vorgeschlagen, wobei der positive prädiktive Wert angibt, mit welcher Wahrscheinlichkeit ein positives Ergebnis auch einer positiven korrekten Diagnose entspricht, der negative Wert, mit welcher Wahrscheinlichkeit ein negatives Ergebnis auch einer negativen korrekten Diagnose entspricht. Diese prädiktiven Werten können wie folgt berechnet werden:

```
PPV= Sensitivität * Prävalenz /
       (Sensitivität * Prävalenz + (1-Sensitivität) * (1-
       Prävalenz))
```

```
NPV= Spezifität * (1-Prävalenz) /
       ((1-Sensitivität) * Prävalenz + Spezifität * (1-
       Prävalenz))
```

Sowohl in den PPV als auch in den NPV geht mithin die Prävalenz ein, wobei bei sinkender Prävalenz der PPV abnimmt, mithin der Untersuchungsvorgang weniger wertvoll wird bzw. Risiken schlechter gerechtfertigt werden.

Für jeden Untersuchungsvorgang kann eine Wahrscheinlichkeitsfunktion angegeben werden, die die Wahrscheinlichkeit einer richtig-positiven (bzw. richtig-negativen, falsch-positiven oder falsch-negativen) Diagnose in Abhängigkeit vom Untersuchungsergebnis (Testergebnis) anzeigt.

Medizinische Diagnosen erfolgen aufgrund der nicht absolut sicheren Aussagen meist in einem mehrstufigem Prozess mit mehreren Untersuchungsvorgängen. Die sei am Beispiel "Lungenkrebs" illustriert. Die Wahrscheinlichkeit einer positiven Diagnose liegt für einen beliebigen Deutschen bei ca. 60/100.000 pro Jahr. Wenn in einem ersten diagnostischen Schritt die Raucheranamnese erhoben wird und die Person als starker Raucher identifiziert wird, steigt das Risiko auf ca. das 30-fache an (1,8/1.000). Diese Wahrscheinlichkeit ist hinreichend, um eine bildgebende Untersuchung, beispielsweise eine Computertomographie, zu rechtfertigen, trotz der damit verbundenen Strahlenbelastung für den Patienten und der entstehenden Kosten. Wird als Untersuchungsergebnis in einem derartigen Untersuchungsvorgang ein Rundherd gefunden, steigt die Wahrscheinlichkeit weiter an, bei einem kleinen Rundherd beispielsweise auf ca. 4/100. Eine derartige Wahrscheinlichkeit rechtfertigt noch keine invasive Behandlungsmaßnahme, aber eine Folgeuntersuchung nach einigen Monaten. Wird in dieser Folgeuntersuchung ein signifikantes Wachstum festgestellt, steigt die Wahrscheinlichkeit auf ca. 2/3. Diese Wahrscheinlichkeit rechtfertigt dann auch eine invasive Maßnahme als Untersuchungsvorgang zur Diagnose, beispielsweise eine Biopsie/Operation, mit der der Diagnosevorgang insgesamt abgeschlossen werden kann.

Auch allgemein kann mithin gesagt werden, dass jeder medizinische Diagnoseprozess als Folge von Wahrscheinlichkeitszuständen gesehen werden kann, in denen jeweils durch eine diagnostische Maßnahme, also einen Untersuchungsvorgang, mit einer bestimmten Wahrscheinlichkeit ein Eintritt in einen anderen Zustand erfolgt. Bei jedem Schritt wird damit von einer Ausgangswahrscheinlichkeit für eine mögliche Gesundheitsanomalie/Krankheit ausgegangen und der Schritt führt zu einer resultierenden Wahrscheinlichkeit für das Vorliegen dieser Gesundheitsanomalie. Zu Beginn ist die Wahrscheinlichkeit der allgemeine Grundwert der Prävalenz in der Population, also für diesen Patienten bzw. eine Personengruppe mit gleichen Untersuchungsvorgängen und Untersuchungsergebnissen.

Derartige Betrachtungen wurden in Zusammenhang mit gesundheitsökonomischen Modellierungen bereits vorgeschlagen, werden jedoch in der klinischen Praxis nicht angewandt. Leitlinien für medizinisches Personal werden in der Regel nicht auf einer derartigen Grundlage erstellt und enthalten in der Regel auch keine Anweisungen, beispielsweise derart, dass ein bestimmter Untersuchungsvorgang nur bei einer bestimmten Ausgangswahrscheinlichkeit (Prävalenz in der Personengruppe, der der Patient angehört) benutzt werden sollte. Dies liegt darin begründet, dass Wahrscheinlichkeiten durch Menschen intuitiv nur schwer erfassbar sind, zum anderen aber auch nicht bekannt sind, so dass eine Anweisung wie "diagnostischen Test nur anfordern, wenn die Wahrscheinlichkeit eines positiven Ergebnisses > 20 % ist" im medizinischen Alltag nicht umsetzbar ist. Hierzu müsste der Arzt für alle Befunde im Kopf haben, welche Wahrscheinlichkeit welches Ergebnis impliziert, was eine kaum zu lösende Aufgabe ist. Mithin werden heute meist grobe Einteilungen, beispielsweise der Framingham-Score beim kardialen Risiko, verwendet.

Auch Leitlinien folgen abgeschätzten Grobschemata. Bei einigen Untersuchungsvorgängen ist bekannt, dass sie nur selten zu einem klinisch signifikanten positiven Ergebnis führen, beispielsweise bei einer Magnetresonanzuntersuchung der Wirbelsäule bei sporadischen Rückenschmerzen, so dass pauschal abgeraten wird. Andere Untersuchungsvorgänge werden in grober Abhängigkeit von Vorbefunden empfohlen, aber ohne genaue Angabe, ab welchem PPV ein Untersuchungsvorgang gerechtfertigt ist.

Derartige Probleme stellen sich insbesondere in der medizinischen Radiologie. In vielen Fällen ist eine klare Diagnose auf Basis einer radiologischen Untersuchung nicht möglich, beispielsweise bei einem mäßig vergrößerten Lymphknoten, sondern es könnte maximal eine Wahrscheinlichkeit abgeschätzt werden. Als Konsequenz werden in radiologischen Befunden bewusst unscharfe Formulierungen verwendet ("nicht auszuschließen"), die von einem Zuweiser als unbefriedigend empfunden werden können. Dabei ist zu beachten, dass bedingte Wahrscheinlichkeiten von Menschen praktisch nicht intuitiv erfassbar sind. So ist die Wahrscheinlichkeit, dass ein vergrößerter Lymphknoten bösartig ist, vom Vorhandensein eines Primärtumors abhängig, jedoch ist medizinisches Personal nicht in der Lage, hier konkrete Wahrscheinlichkeiten anzugeben, die aber nötig wären, um invasive Maßnahmen zu planen.

Auch außerhalb der Radiologie existieren derartige Probleme jedoch. Beispielsweise werden in der Labordiagnostik relativ willkürlich Grenzwerte für Laborwerte festgelegt, obwohl die Wahrscheinlichkeit einer positiven Diagnose kontinuierlich mit dem Laborwert steigt. Daher kann verlässlich für jeden konkreten Laborwert nur eine Wahrscheinlichkeit angegeben werden, jeglicher Grenzwert ist immer künstlich festgelegt. Dennoch wird heute häufig bei Überschreitung eines Grenzwerts von einer positiven Diagnose ausgegangen.

Insgesamt bleibt festzuhalten, dass ein diagnostischer Untersuchungsvorgang, der eine klare Diagnose erbringt, eher eine Ausnahme als die Regel ist. In der Mehrheit der Fälle wird nur eine Ausgangswahrscheinlichkeit für eine Krankheit (Prävalenz) in eine (höhere oder niedrigere) Wahrscheinlichkeit für die Gesundheitsanomalie geändert. Dies geschieht in der heutigen medizinischen Praxis rein qualitativ und nicht quantitativ, was zu einer hohen Menge an Redundanz und somit zusätzlichen Kosten führt.

Der Erfindung liegt daher die Aufgabe zugrunde, eine Möglichkeit zur verbesserten Unterstützung von medizinischem Personal anzugeben, die die Kenntnis über die naturwissenschaftlich gegebene Aussagekraft von Untersuchungsergebnissen, die insbesondere durch Messung gewonnen werden, ausnutzt.

Zur Lösung dieser Aufgabe sind erfindungsgemäß ein Verfahren mit den Merkmalen des Anspruchs 1, ein Unterstützungssystem mit den Merkmalen des Anspruchs 13, ein Computerprogramm mit den Merkmalen des Anspruchs 14 und ein elektronisch lesbarer Datenträger mit den Merkmalen des Anspruchs 15 vorgesehen. Vorteilhafte Ausgestaltungen der Erfindung ergeben sich aus den abhängigen Ansprüchen.

Ein erfindungsgemäßes Verfahren zur Unterstützung von medizinischem Personal bei der Untersuchung eines Patienten mittels wenigstens eines insbesondere eine Messung umfassenden Untersuchungsvorgangs sieht mithin vor, dass unter Berücksichtigung von in einer ersten Datenbank vorliegenden Patientendaten, die wenigstens eine Eigenschaft des Patienten und/oder wenigstens ein Untersuchungsergebnis wenigstens eines bereits abgeschlossenen Untersuchungsvorgangs umfassen, einer elektronischen Patientenakte wenigstens ein jeweils auf eine zu stellende Diagnose bezogener Prävalenzwert für eine den Patienten umfassende Personengruppe, zu der gehörig der Patient in Abhängigkeit wenigstens eines Teils der Patientendaten klassifiziert wurde, ermittelt wird und aus dem Prävalenzwert wenigstens eine auf einen hinsichtlich der zu stellenden Diagnose durchführbaren, zukünftigen Untersuchungsvorgang bezogene Bewertungsinformation unter Berücksichtigung von in einer zweiten Datenbank vorliegenden, die Sensitivität und/oder die Spezifität für den wenigstens einen zukünftigen Untersuchungsvorgang hinsichtlich der zu stellenden Diagnose beschreibenden Prädiktionsinformationen erzeugt und an das medizinische Personal ausgegeben wird.

Erfindungsgemäß wurde also erkannt, dass eine Auswertung der hochkomplexen, intuitiv für den Menschen schwer erfassbaren physikalisch-technischen Zusammenhänge im Rahmen der Untersuchungsvorgänge/diagnostischen Tests durch ein geeignetes Rechnersystem erstmals ermöglicht wird. Durch Messungen verschiedenster Art erzielte Ergebnisse, mithin grundlegende physikalisch-technische Sachverhalte im Diagnosevorgang, der letztlich ein Messvorgang ist, werden mit technischen Mitteln zusammengeführt, um einen Mehrwert im Hinblick auf den weiteren Verlauf des gesamten Diagnoseprozesses für medizinisches Personal bereitzustellen. Genutzt wird hierbei insbesondere ein Kontext, der ohnehin in der technischen Realisierung voranschreitet, nämlich die sogenannte elektronische Patientenakte, in der Patientendaten, sowohl was Eigenschaften des Patienten angeht, insbesondere hinsichtlich einer Anamnese, als auch durchgeführte Untersuchungsvorgänge und deren Ergebnisse als Patientendaten vorgehalten werden können. Zunächst wird ein Prävalenzwert basierend auf den Patientendaten, die die Personengruppe, der der Patient angehört, definieren, ermittelt, das bedeutet, eine patientenbezogene Wahrscheinlichkeit, dass eine bestimmte Gesundheitsanomalie, insbesondere Krankheit, gegeben ist, wobei es zu dessen Ermittlung zwei grundsätzliche Möglichkeiten gibt, wie im Folgenden noch genauer dargestellt werden wird. Dabei wird es im Übrigen bevorzugt, Prävalenzwerte für verschiedene zu stellende Diagnosen, also verschiedene Gesundheitsanomalien, immer aktuell in der Patientenakte vorzuhalten, so dass der Prävalenzwert nach seiner Ermittlung letztlich zu einem Teil der Patientendaten wird und dort grundsätzlich verfügbar ist, wenn Unterstützung für neue Untersuchungsvorgänge gegeben werden soll. Selbstverständlich ist hierbei der Prävalenzwert ständig aktuell zu halten, das bedeutet, neue Untersuchungsvorgänge und Untersuchungsergebnisse, die, wie eingangs dargelegt, die Wahrscheinlichkeiten verschieben, sind zu berücksichtigen.

Die erste und die zweite Datenbank können zusammengefasst werden, insbesondere auf derselben Recheneinrichtung und/oder in derselben Cloud vorliegen; denkbar ist es jedoch auch, die erste Datenbank und/oder die zweite Datenbank aufzuteilen, beispielsweise als verteiltes System und/oder innerhalb einer Cloud auf unterschiedlichen Recheneinrichtungen zu realisieren und/oder sogar die elektronischen Patientenakten beispielsweise immer innerhalb der medizinischen Einrichtungen zu speichern, wo der Patient zur Zeit in Behandlung ist.

Wie bereits dargelegt wurde, bezieht sich die zu stellende Diagnose insbesondere auf eine spezifische Gesundheitsanomalie bzw. Gesundheitsstörung, insbesondere eine Krankheit, hat also als Ziel, das Vorliegen bzw. das Nichtvorliegen der Gesundheitsanomalie möglichst verlässlich anzugeben. Entsprechend ist es auch zweckmäßig, wenn, wie angemerkt, aktuelle Prävalenzwerte für die Patienten innerhalb der elektronischen Patientenakten gespeichert werden, da dann für verschiedene zu stellende Diagnosen bereits ermittelte Prävalenzwerte bereitstehen, beispielsweise im Rahmen von Vorsorgeuntersuchungen für verschiedene typische Krankheiten. Dabei sei im Übrigen angemerkt, dass ein Untersuchungsvorgahng, auch wenn er im Hinblick auf eine bestimmte Gesundheitsanomalie durchgeführt wird, durchaus Einfluss auf mehrere Prävalenzwerte haben kann.

Zusammenfassend wird in einem Unterstützungssystem mithin eine Vielzahl verschiedener Auswertungsergebnisse, die Wissen über die Prävalenz von Krankheiten und die messtechnische Funktionsweise von Untersuchungsvorgängen zusammenfassen, analysiert, um medizinisches Personal bei noch durchzuführenden Untersuchungsvorgängen so zu unterstützen, so dass eine optimale Untersuchung des Patienten in bevorzugt mehrerlei Hinsicht ermöglicht wird. Automatisiert und im Hintergrund werden für Menschen schwer intuitiv erfassbare Konzepte der physikalisch-technischen Zusammenhänge analysiert und ausgewertet, insbesondere hinsichtlich verketteter Wahrscheinlichkeiten und/oder einer Modellierung von Patientenzuständen hinsichtlich zu diagnostizierender Gesundheitsanomalien, um die für Menschen nützliche Bewertungsinformation bereitzustellen und die Behandlung des Patienten, insbesondere hinsichtlich einer möglichst verlässlichen Diagnosestellung und/oder einer optimalen Ausnutzung der technischen Möglichkeiten, zu verbessern.

In einer zweckmäßigen Ausgestaltung können Prädiktionsinformationen in Form eines Sensitivitätswertes und eines Spezifitätswertes und/oder einer ROC-Kurve verwendet werden. Dabei ist es bevorzugt, als Wahrscheinlichkeitsfunktionen sogenannte ROC-Kurven der Untersuchungsvorgänge/diagnostischen Tests einzusetzen, die beispielsweise aus Studien bekannt sein können. ROC-Kurven (Receiver-Operating-Characteristic-Kurven), oft auch als Grenzwertoptimierungskurve bezeichnet, sind eine Methode zur Bewertung und Optimierung von Analysestrategien. Dabei können beispielsweise für mögliche Parameterwerte von sowohl Eingangsdaten als auch Untersuchungsergebnisse resultierende relative Häufigkeitsverteilungen in Form von Sensitivität (Richtig-Positiv-Rate) und Spezifität angegeben werden. Damit existiert in Form der Prädiktionsinformation insbesondere eine hervorragende Grundlage, um beispielsweise prädiktive Werte, also insbesondere den positiven prädiktiven Wert (PPV) und den negativen prädiktiven Wert (NPV), zu ermitteln.

Zur Ermittlung des Prävalenzwertes gibt es im Wesentlichen zwei Ansatzmöglichkeiten, die auch kombiniert verwendet werden können.

So kann zum einen vorgesehen sein, dass der Prävalenzwert zumindest teilweise ausgehend von einem Grundwert für eine durch Patienteneigenschaften definierte Personengruppe unter Berücksichtigung vorliegender Untersuchungsergebnisse und der den bereits abgeschlossenen Untersuchungsvorgang zugeordneten Prädiktionsinformation bestimmt wird. Auf diese Weise ist es mithin möglich, den Patienten rückschauend zu modellieren. Ausgangspunkt ist ein Grundwert für eine Personengruppe der Bevölkerung, der der Patient angehört und die anhand von Patienteneigenschaften bestimmt werden kann. Patienteneigenschaften können hierbei eine Vielzahl von Informationen beschreiben, beispielsweise das Geschlecht des Patienten, Informationen zur Statur des Patienten (beispielsweise Body-Mass-Index), zu Gewohnheiten des Patienten (Rauchen etc.), zum Alter des Patienten und/oder zur Einordnung in Risikogruppen. Patienteneigenschaften können mithin auch Anamneseergebnisse enthalten. Für die jeweiligen Personengruppen sind nun, beispielsweise anhand einer dritten Datenbank, Prävalenzwerte verfügbar oder ermittelbar, die die allgemeine Wahrscheinlichkeit für das Vorliegen einer Gesundheitsanomalie angeben. Geht man von diesem Grundwert aus, wird die Wahrscheinlichkeit durch Untersuchungsergebnisse bereits erfolgter Untersuchungsvorgänge, die sich auf die zu stellende Diagnose beziehen, modifiziert. Nachdem durch die Prädiktionsinformation die Art und Weise bekannt ist, wie sich die Wahrscheinlichkeiten verschieben, lässt sich mithin, insbesondere anhand von Wahrscheinlichkeitsmodellen, die Wahrscheinlichkeitsketten beschreiben, eine aktuelle Wahrscheinlichkeit, dass der Patient die Gesundheitsanomalie aufweist, und somit ein aktueller Prävalenzwert ermitteln.

Im Rahmen der vorliegenden Erfindung ist es jedoch auch mit besonderem Vorteil möglich, die Vielzahl von Daten und Untersuchungsergebnissen, die durch die Vielzahl an elektronischen Patientenakten unterschiedlicher Patienten gegeben ist, auszunutzen, um auf diese Art und Weise Prävalenzwerte zu ermitteln. So kann vorgesehen sein, dass der Grundwert und/oder der Prävalenzwert für die jeweilige Personengruppe wenigstens teilweise in Abhängigkeit von Patientendaten von weiteren, in der ersten Datenbank erfassten, der jeweiligen Personengruppe zugehörigen Patienten, zu denen eine Grundwahrheit bezüglich der zu stellenden Diagnose bekannt oder ableitbar ist, ermittelt wird. Spätestens mit dem Tod eines Patienten und/oder einer hochgradig sichergestellten Diagnose wird bekannt, ob die Gesundheitsanomalie bei dem Patienten tatsächlich vorliegt bzw. vorlag, so dass eine Grundwahrheit existiert. Nachdem es schwer ist, das Nichtvorliegen einer Gesundheitsanomalie sicher nachzuweisen, können entsprechende, insbesondere zeitliche, Bedingungen geschaffen werden, unter denen davon ausgegangen werden kann, dass eine Gesundheitsanomalie bei einem Patienten nicht vorliegt, was ebenso als Grundwahrheit aufgefasst werden kann. Beispielsweise kann vorgesehen sein, dass bei weiteren Patienten, für die ein auf die zu stellende Diagnose bezogene Untersuchungsvorgang bereits durchgeführt wurde, der keine Grundwahrheit liefert, bei einer innerhalb einer vordefinierten Zeitspanne nicht aufgetretenen weiteren Untersuchung und/oder einen nicht innerhalb der Zeitspanne erbrachten Nachweis für das Vorliegen der spezifischen Gesundheitsanomalie, auf die sich die zu stellende Diagnose bezieht, von einer negativen Diagnose als Grundwahrheit ausgegangen wird. Wurde beispielsweise vor zwei Jahren ein bestimmter diagnostischer Test als Untersuchungsvorgang durchführt, der Patient aber bislang weder weiter untersucht noch als positiv markiert, ist davon auszugehen, dass der Patient nicht erkrankt ist. Auch bei Patienten, die nie in Bezug auf die Gesundheitsanomalie auffällig wurden, kann von einer negativen Diagnose als Grundwahrheit ausgegangen werden.

Derartige Grundwahrheiten, die aus der ersten Datenbank selbst bestimmbar sind, können gemeinsam mit den Patientendaten nun bei hinreichend gut durch die elektronischen Patientenakten bestimmten Personengruppen genutzt werden, um Prävalenzwerte für in den Patientendaten entsprechend übereinstimmende Patienten aus der ersten Datenbank und somit den Patientenakten selbst zu bestimmen. Auf diese Weise findet mithin eine weitere Auswertung physikalischer Sachverhalte statt, die nutzbringend zur Ermittlung von Bewertungsinformationen für medizinisches Personal eingesetzt wird.

Dabei sei jedoch hervorgehoben, dass derartige Grundwahrheiten (ground truth) selbstverständlich auch zur Ermittlung von Prädiktionsinformationen aus der ersten Datenbank abgeleitet werden können. So kann vorgesehen sein, dass die Prädiktionsinformation für einen Untersuchungsvorgang wenigstens teilweise in Abhängigkeit von Patientendaten von weiteren in der ersten Datenbank erfassten, dem Untersuchungsvorgang unterzogenen Patienten, zu denen eine Grundwahrheit bezüglich der zu stellenden Diagnose bekannt oder ableitbar ist, ermittelt wird. Sind mithin beispielsweise für bestimmte Untersuchungsvorgänge die Prädiktionsinformationen, insbesondere Wahrscheinlichkeitsinformationen, nicht bereits aus wissenschaftlichen Studien oder anderweitig bekannt, kann dann, wenn eine große Zahl an elektronischen Patientenakten bereitsteht, mittels der ersten Datenbank eine Ermittlung der entsprechenden Prädiktionsinformationen erfolgen. Immer dann, wenn definitive Diagnosen, also Grundwahrheiten, für Patienten vorliegen, lassen sich beispielsweise Wahrscheinlichkeitsfunktionen und/oder andere Prädiktionsinformationen für diagnostische Tests, denen noch keine Prädiktionsinformation in der zweiten Datenbank zugeordnet ist, rückwirkend einfach berechnen. Mithin werden Informationen über die Aussagekraft von physikalischen Untersuchungsergebnissen vorteilhaft aus einer Vielzahl verfügbarer Daten abgeleitet, um Prädiktionsinformationen bereitzustellen, die zur Ermittlung von Prävalenzwerten und/oder Bewertungsinformationen herangezogen werden können.

Es lässt sich eine Vielzahl von konkreten Funktionen eines derart beschriebenen Unterstützungssystems realisieren. Dabei sei an dieser Stelle nochmals angemerkt, dass es sich bei dem Prävalenzwert genauso wenig wie bei der Bewertungsinformation um eine eigentliche Diagnose oder dergleichen handelt, sondern lediglich um einen Rechenwert, der statistische und gegebenenfalls physikalisch-technische Zusammenhänge beschreibt. Die noch zu stellende Diagnose bleibt vollständig durch Begutachtung verschiedener Untersuchungsergebnisse und entsprechende Schlussfolgerungen dem medizinischen Personal, insbesondere dem Arzt, vorbehalten. Durch die Bewertungsinformation werden lediglich Wege aufgezeigt, möglichst sicher und/oder effizient an die nötigen Informationen für die eigentliche Stellung einer Diagnose zu gelangen.

Es existieren eine Vielzahl von Möglichkeiten zur konkreten Unterstützung von medizinischem Personal. So kann beispielsweise vorgesehen sein, dass eine Bewertungsinformation für wenigstens einen zukünftigen Untersuchungsvorgang automatisch erzeugt wird, wenn ein Prävalenzwert einen insbesondere für die zu stellende Diagnose spezifischen Schwellwert überschreitet. Oft liegen Schwellwerte vor, ab denen ein Untersuchungsvorgang als sinnvoll angesehen werden kann und/oder eine Kostenerstattung oder dergleichen ermöglicht wird. Solche Schwellwerte können eingesetzt werden, um beispielsweise bei Aufruf der elektronischen Patientenakte eines Patienten, wenn die Schwellwerte überschritten sind, den entsprechenden Untersuchungsvorgang automatisiert vorzuschlagen. Ist der Prävalenzwert beispielsweise größer als eine bestimmte Prozentzahl oder ergibt sich unter Berücksichtigung des aktuellen Prävalenzwertes ein positiver prädiktiver Wert (PPV) von größer einem bestimmten Prozentsatz, kann der entsprechende diagnostische Test vorgeschlagen werden und das Untersuchungsergebnis wiederum berücksichtigt werden, um aktualisierte Prävalenzwerte zu ermitteln und dergleichen, bis es möglich ist, die Diagnose als gesichert zu markieren, insbesondere durch das medizinische Personal. Eine Empfehlung eines Untersuchungsvorgangs kann auch dann erfolgen, wenn ein neues Patientendatum ermittelt und/oder eingegeben worden ist. Beispielsweise kann bei einer Raucher-Anamnese eine deutliche Steigerung des Prävalenzwerts für Lungenkrebs erwartet werden. Ist der Patient auch fortgeschrittenen Alters, kann ein entsprechender Schwellwert zur Empfehlung bestimmter Untersuchungsvorgänge überschritten sein. Entsprechende Untersuchungsvorgänge können als Bewertungsinformation dann empfohlen werden.

Die Bewertungsinformation kann insbesondere für einen benutzerseitig ausgewählten Untersuchungsvorgang bestimmt werden. Beispielsweise anhand der eingangs genannten Formeln ist es bei einem vorliegenden aktuellen Prävalenzwert und Kenntnis der Prädiktionsinformationen, insbesondere was Sensitivität und/oder Spezifität angeht, leicht möglich, für den Patienten bzw. die entsprechende Personengruppe, der der Patient angehört, zutreffende prädiktive Werte, insbesondere PPV und NPV, zu ermitteln. Dies ist beispielsweise dann zweckmäßig, wenn ein durchzuführender Untersuchungsvorgang durch medizinisches Personal bei geöffneter elektronischer Patientenakte ausgewählt wurde. Beispielsweise kann dann eine Ausgabe in der Art "Bei einem positiven Testergebnis ist der Patient mit 83 % Wahrscheinlichkeit wirklich krank, bei einem negativen mit 87 % wirklich gesund - wollen Sie den Test durchführen?" erfolgen. Insbesondere kann sich medizinisches Personal auf diese Art und Weise für unterschiedliche mögliche Untersuchungsvorgänge prädiktive Werte anzeigen lassen und, beispielsweise im Hinblick darauf, ob ein Ausschluss oder eine Bestätigung stattfinden soll, beurteilen, ob der Untersuchungsvorgang tatsächlich durchgeführt werden soll.

Wie bereits angedeutet wurde, kann die Bewertungsinformation bevorzugt wenigstens einen empfohlenen Untersuchungsvorgang, insbesondere eine Reihe empfohlener Untersuchungsvorgänge, umfassend ermittelt werden. Gerade jedoch im Hinblick auf eine komplette weitere Untersuchungsstrategie, mithin eine Reihenfolge weiterer Untersuchungsvorgänge, erweist sich das erfindungsgemäße Unterstützungsverfahren als besonders vorteilhaft. Konkret kann hierbei vorgesehen sein, dass als Bewertungsinformation eine wenigstens einen Untersuchungsvorgang umfassende Reihenfolge von Untersuchungsvorgängen in einem Optimierungsverfahren ermittelt wird, wobei die Optimierung hinsichtlich einer Zuverlässigkeit einer positiven Diagnose und/oder einer Zuverlässigkeit einer negativen Diagnose und/oder der Kostengünstigkeit und/oder des Zeitbedarfs für die Untersuchungsvorgänge erfolgt und/oder wenigstens eine auf eine minimale Zuverlässigkeit abzielende Randbedingung verwendet wird. Es wird mithin ermöglicht, die im Hinblick auf unterschiedliche Aspekte günstigste und geeignetste Reihenfolge weiterer Untersuchungsvorgänge hinsichtlich der zu stellenden Diagnose zu ermitteln. Beispielsweise kann das kostengünstigste und/oder schnellste weitere Vorgehen aufgefunden werden, welches dennoch (als Randbedingung) eine hinreichende Sicherheit der abschließenden insgesamten Diagnose bereitstellt, beispielsweise einen nach dem letzten Untersuchungsvorgang mindestens gegebenen PPV und/oder NPV. Derartige Funktionen können beispielsweise durch einen Benutzer gezielt angewählt werden, beispielsweise unter Nutzung eines Benutzerinterfaces, welches Zugriff auf das erfindungsgemäße Unterstützungssystem liefert. Hierbei können Simulationen und/oder mathematische Wahrscheinlichkeitsmodelle bevorzugt eingesetzt werden, beispielsweise eine Simulation der Entwicklung von PPV und NPV über verschiedene Pfade, mithin unterschiedliche Reihenfolgen von verschiedenen Untersuchungsvorgängen. Dabei können besonders vorteilhaft auch gesundheitsökonomische Parameter, beispielsweise Kosten der diagnostischen Tests, eingehen.

Insgesamt ist es, wie bereits dargestellt wurde, besonders bevorzugt, wenn zur Ermittlung der Bewertungsinformation (und gegebenenfalls für die Ermittlung des Prävalenzwerts unter Nutzung von Prädiktionsinformationen) der zukünftige (bzw. bereits erfolgte) Untersuchungsvorgang in einem mathematischen Wahrscheinlichkeitsmodell berechnet und/oder simuliert wird. Mit besonderem Vorteil können hierbei als Wahrscheinlichkeitsmodell ein Bayessches Netz und/oder eine Markov-Kette verwendet werden und/oder die Simulation eine Monte-Carlo-Simulation umfassen. Es können also geeignete mathematische Verfahren für eine Modellierung der Kette an diagnostischen Testes eingesetzt werden, die insbesondere auch in der Statistik Verwendung finden, insbesondere Bayessche Netze und/oder Markov-Ketten. Eine Simulation kann hierbei insbesondere hinsichtlich der Entwicklung von PPV und NPV über mehrere Untersuchungsvorgänge erfolgen, beispielsweise unter Nutzung einer Monte-Carlo-Simulation.

Eine weitere Funktion, die die Erfindung bereitstellen kann, sieht vor, dass die Bewertungsinformation eine Auflistung von Untersuchungsvorgängen umfasst, für die der Prävalenzwert einen deren Zulässigkeit beschreibenden Grenzwert überschreitet. Insbesondere dann, wenn also Vorgaben existieren, die erfüllt sein müssen, damit bestimmte Untersuchungsvorgänge eingesetzt werden können, kann eine zweckmäßige Funktionalität die Auflistung zulässiger weiterer diagnostischer Tests sein.

Eine weitere vorteilhafte Ausgestaltung der vorliegenden Erfindung sieht vor, dass bei der Ermittlung der Bewertungsinformation wenigstens eine Verfügbarkeitsinformation bezüglich des wenigstens einen zukünftigen Untersuchungsvorgangs, insbesondere aus einem Informationssystem abgerufen, berücksichtigt wird. Zweckmäßig ist es dabei, wenn das erfindungsgemäße Unterstützungssystem in ein Informationssystem, beispielsweise ein Krankenhausinformationssystem und/oder ein Radiologieinformationssystem, wenigstens teilweise integriert wird. In jedem Fall kann zweckmäßig vorgesehen sein, einen Austausch mit einem derartigen Informationssystem vorzunehmen, um auch die Verfügbarkeit von Untersuchungsvorgängen zu überprüfen und entsprechend bei der Ermittlung der Bewertungsinformation zu berücksichtigen. Eine temporäre Nichtverfügbarkeit eines Untersuchungsvorgangs kann beispielsweise gegeben sein, wenn ein entsprechendes Untersuchungsgerät zu stark ausgelastet ist; es ist auch eine andauernde Nichtverfügbarkeit denkbar, wenn eine medizinische Einrichtung, an der das Unterstützungsverfahren ausgeführt wird, das notwendige Untersuchungsgerät nicht aufweist und dergleichen. Eine Kopplung zwischen einem Informationssystem und einem Unterstützungssystem der erfindungsgemäßen Art ermöglicht also eine weitergehende, auf die Situation des medizinischen Personals maßgeschneiderte Bewertung möglicher zukünftiger Untersuchungsvorgänge.

In einer Weiterbildung des erfindungsgemäßen Verfahrens kann auch vorgesehen sein, dass bei einem mittels eines medizinisch-technischen Untersuchungsgeräts durchzuführenden zukünftigen Untersuchungsvorgang ein auf die Personengruppe abgestimmtes Untersuchungsprotokoll als Bewertungsinformation ermittelt und an das Untersuchungsgerät übermittelt und/oder dort ausgeführt wird und/oder wenigstens die Anzeige der Bewertungsinformation an dem Untersuchungsgerät erfolgt. Nachdem im Unterstützungssystem die diagnostische Fragestellung bereits bekannt ist, ist es mithin möglich, geeignete Untersuchungsprotokolle für bestmögliche Untersuchungsergebnisse auch bereits seitens des Unterstützungssystems auszuwählen und insbesondere gleich dem Untersuchungsgerät bereitzustellen, beispielsweise Bildaufnahmeprotokolle. Diese Untersuchungsprotokolle können in bevorzugter Ausgestaltung noch in Abhängigkeit der Patientendaten angepasst werden, beispielsweise für den Patienten optimiert werden. Dies vereinfacht die Arbeit des medizinischen Personals weiter und unterstützt dieses auch im Hinblick auf eine möglichst weitgehend automatisierte Durchführung des zukünftigen Untersuchungsvorgangs. Insbesondere ist es im Rahmen der vorliegenden Erfindung auch möglich, an einem Untersuchungsgerät selbst Anfragen an das Unterstützungssystem zu stellen und eine entsprechende Anzeige von Bewertungsinformationen zu erhalten. Beispielsweise kann seitens des Untersuchungsgeräts ein Softwaremittel vorgesehen sein, welches zur Kommunikation mit weiteren Komponenten des Unterstützungssystems ausgebildet ist und entsprechende Anfragen weiterleitet sowie die Bewertungsinformationen entgegennimmt. Das Konzept des verteilten Unterstützungssystems, insbesondere unter Verwendung der sogenannten "Apps", lässt sich selbstverständlich auch erweitern, beispielsweise auf von medizinischem Personal mitgeführte Mobilgeräte und dergleichen. Denkbar ist es ferner auch, einen Zugriff auf das Unterstützungssystem über ein Zugriffsportal, insbesondere über das Internet, bereitzustellen.

Neben dem erfindungsgemäßen Verfahren betrifft die Erfindung auch ein Unterstützungssystem, welches mithin insbesondere aufweist:

- eine erste Datenbank mit elektronischen Patientenakten mehrerer Patienten, die jeweils Patientendaten mit wenigstens einer Eigenschaft des Patienten und/oder wenigstens einem Untersuchungsergebnis wenigstens eines bereits abgeschlossenen Untersuchungsvorgangs an dem Patienten enthalten,
- eine zweite Datenbank mit die Sensitivität und/oder die Spezifität für jeweils einen Untersuchungsvorgang hinsichtlich jeweils einer zu stellenden Diagnose beschreibenden Prädiktionsinformationen,
- eine Klassifizierungseinheit zur Zuordnung eines Patienten zu einer Personengruppe in Abhängigkeit wenigstens eines Teils der Patientendaten des Patienten und einer zu stellenden Diagnose,
- eine Prävalenzermittlungseinheit zur Ermittlung eines auf die zu stellende Diagnose bezogenen Prävalenzwerts für den Patienten in Abhängigkeit der Personengruppe und/oder der Patientendaten,
- eine Bewertungsinformationsermittlungseinheit zur Ermittlung einer auf wenigstens einen hinsichtlich der zu stellenden Diagnose durchführbaren, zukünftigen Untersuchungsvorgang bezogenen Bewertungsinformation unter Berücksichtigung des Prävalenzwerts für die zu stellende Diagnose und den Patienten und der Prädiktionsinformationen für den wenigstens einen Untersuchungsvorgang, und
- eine Ausgabeeinheit für die Bewertungsinformation.

Sämtliche Ausführungen bezüglich des erfindungsgemäßen Verfahrens gelten für das erfindungsgemäße Unterstützungssystem entsprechend fort. Insbesondere kann die Prävalenzermittlungseinheit, wie dargelegt, alternativ oder kombiniert Prävalenzwerte ausgehend von einem Grundwert für eine Ausgangspersonengruppe ermitteln oder aber den Inhalt der ersten Datenbank entsprechend auswerten. Die Klassifizierungseinheit kann also beispielsweise ausgehend von Patienteneigenschaften den Patienten einer Personengruppe zuordnen, für die, beispielsweise in einer dritten Datenbank, ein Grundwert für die Prävalenz vorliegt, wobei dann die Prävalenzermittlungseinheit anhand bereits vorgenommener Untersuchungsvorgänge hieraus einen aktuellen Prävalenzwert ableiten kann, wenn die Prädiktionsinformationen für die bereits erfolgten Untersuchungsvorgänge vorliegen. In einer solchen dritten Datenbank können selbstverständlich auch für Personengruppen, die auch anhand von Untersuchungsergebnissen für erfolgte Untersuchungsvorgänge klassifiziert sind, Prävalenzwerte abgelegt sein; solche Prävalenzwerte können schließlich auch, wie bereits bezüglich des Verfahrens dargelegt wurde, aus Patientendaten der ersten Datenbank abgeleitet werden.

Ein erfindungsgemäßes Unterstützungssystem kann auch weitere Einheiten und Subeinheiten umfassen und kann insbesondere, zumindest teilweise, in einer Cloud und/oder als Teil einer Cloud realisiert werden. Eine Ankopplung an externe Informationssysteme ist ebenso möglich. Weitere Funktionseinheiten des erfindungsgemäßen Unterstützungssystems können beispielsweise Auswertungseinheiten zur Bestimmung von Grundwerten und/oder Prävalenzwerten und/oder Prädiktionsinformationen auf Basis der Patientendaten sein. Das Unterstützungssystem kann ferner auch Softwaremittel umfassen, die beispielsweise auf Mobilgeräten und/oder Untersuchungsgeräten vorhanden sein können, um eine Benutzerschnittstelle zu dem Unterstützungssystem bereitzustellen.

Ein erfindungsgemäßes Computerprogramm ist beispielsweise direkt in einen Speicher einer Recheneinrichtung ladbar und weist Programmmittel auf, um die Schritte eines hierin beschriebenen Verfahrens auszuführen, wenn das Computerprogramm in der Recheneinrichtung ausgeführt wird. Das Computerprogramm kann auf einem erfindungsgemäßen elektronisch lesbaren Datenträger gespeichert sein, welcher mithin darauf gespeicherte elektronisch lesbare Steuerinformationen umfasst, welche zumindest ein genanntes Computerprogramm umfassen und derart ausgestaltet sind, dass sie bei Verwendung des Datenträgers in einer Recheneinrichtung ein hierin beschriebenes Verfahren durchführen. Der Datenträger ist bevorzugt ein nichttransienter Datenträger, beispielsweise eine CD-ROM.

Weitere Vorteile und Einzelheiten der vorliegenden Erfindung ergeben sich aus den im Folgenden beschriebenen Ausführungsbeispielen sowie anhand der Zeichnungen. Dabei zeigen:

Fig. 1    eine Prinzipskizze eines erfindungsgemäßen Untersuchungssystems,

Fig. 2    einen Ablaufplan eines Ausführungsbeispiels des erfindungsgemäßen Verfahrens, und

Fig. 3    eine Zeichnung zur Modellierung von Wahrscheinlichkeitsentwicklungen durch Untersuchungsvorgänge.

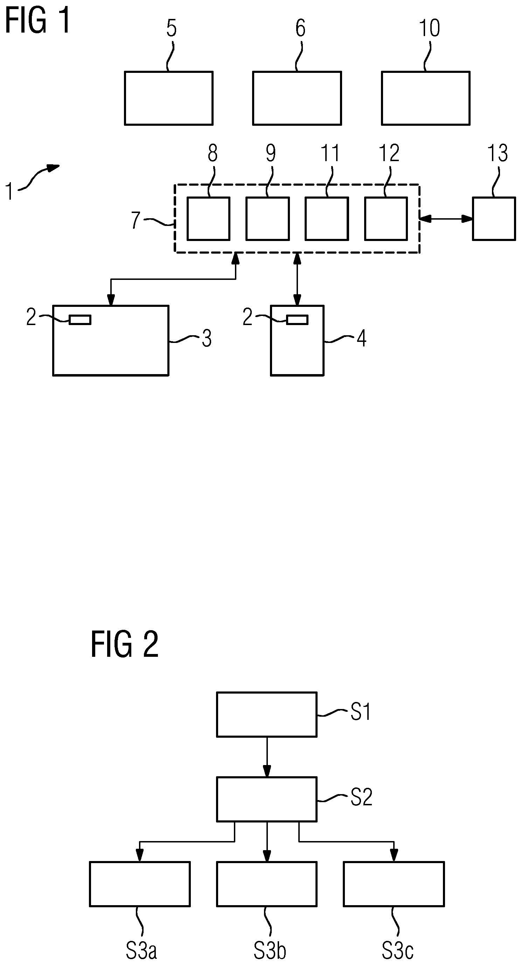

Fig. 1 zeigt eine Prinzipskizze eines erfindungsgemäßen Unterstützungssystems 1. Das Unterstützungssystem 1 wird vorliegend durch wenigstens eine Recheneinrichtung, üblicherweise mehrere Recheneinrichtungen, realisiert, die wiederum wenigstens teilweise Teil einer Cloud darstellen können. Über geeignete Schnittstellenmittel 2, beispielsweis sogenannte "Apps" als Softwaremittel und/oder Internetportale, kann das Unterstützungssystem 1 mit Untersuchungsgeräten 3 zur Durchführung eines Untersuchungsvorgangs, Mobilgeräten 4, die beispielsweise medizinischem Person zugeordnet sein können, sowie weiteren externen und/oder nur teilweise genutzten Recheneinrichtungen kommunizieren.

Das Unterstützungssystem 1 weist eine erste Datenbank 5 auf, in der Patientendaten unterschiedlicher Patienten in Form von elektronischen Patientenakten gespeichert sind. Die Patientendaten umfassen dabei sowohl Eigenschaften des Patienten, wie auch bereits erfolgte Untersuchungsvorgänge sowie deren Untersuchungsergebnisse. Als Teil der Patientendaten sind ferner Prävalenzwerte für verschiedene zu stellende Diagnosen, also verschiedene spezifische Gesundheitsanomalien, insbesondere Krankheiten, in den elektronischen Patientenakten gespeichert, deren Ermittlung und Aktualisierung im Folgenden noch genauer diskutiert werden wird.

Zur Ermittlung von Prävalenzwerten für Patienten weist die wenigstens eine Recheneinrichtung 7 des Unterstützungssystems 1 eine Klassifizierungseinheit 8 und eine Prävalenzermittlungseinheit 9 auf. Mittels der Klassifizierungseinheit 8 können die Patientendaten der ersten Datenbank 5 ausgewertet werden, um den Patienten wenigstens einer Personengruppe zuzuordnen, die bei der Ermittlung bzw. Aktualisierung von Prävalenzwerten mittels der Prävalenzermittlungseinheit 9 berücksichtigt wird. Dabei sind zwei grundsätzliche, auch kumulativ einsetzbare Möglichkeiten denkbar, Prävalenzwerte für bestimmte zu stellende Diagnosen für einen Patienten mittels der Prävalenzermittlungseinheit 9 zu ermitteln. Zum einen ist es möglich, dass die Personengruppen auch bereits durchgeführte Untersuchungsvorgänge und deren Untersuchungsergebnisse berücksichtigen, wobei dann den entsprechenden Personengruppen auch die durchgeführten Untersuchungsvorgänge berücksichtigende Prävalenzwerte unmittelbar zugeordnet werden können, die beispielsweise aus einer optionalen dritten Datenbank 10 abgerufen werden können und/oder aus Patientendaten der ersten Datenbank 5 abgeleitet werden können, wobei für diese Patienten dann bereits eine Grundwahrheit hinsichtlich der zu stellenden Diagnose vorliegt, mithin eine als gesichert geltende Diagnosestellung für diese weiteren Patienten in deren Patientendaten vorliegt, die beispielsweise auf einer abschließenden Beurteilung durch medizinisches Personal basieren kann oder aber auch daraus gefolgert werden kann, dass für eine bestimmte Zeitspanne, beispielsweise ab einem bestimmten Untersuchungsvorgang, keine weiteren Einträge hinsichtlich der Gesundheitsanomalie vorliegen, so dass davon ausgegangen werden kann, dass die Gesundheitsanomalie bei dem Patienten nicht vorliegt. Mit steigender Anzahl in der ersten Datenbank 5 erfasster Patienten steigt die Genauigkeit einer derartigen Bestimmung eines Prävalenzwertes.

Denkbar ist es aber auch, von einem insbesondere wiederum in der optionalen Datenbank 10 gespeicherten Grundwert für eine Personengruppe, der der Patient angehört, auszugehen, und die Entwicklung der Prävalenz aufgrund bereits durchgeführter Untersuchungsvorgänge und deren Sensitivität/Spezifität zu modellieren, insbesondere anhand eines Wahrscheinlichkeitsmodells. Auch die erwähnten Grundwerte lassen sich dabei selbstverständlich bei hinreichend vorliegenden Grundwahrheiten bezüglich weiterer Patienten aus den Patientendaten der erste Datenbank 5 ableiten.

Prädiktionsinformationen bezüglich verschiedener Untersuchungsvorgänge, die deren Sensitivität und Spezifität beschreiben, sind in einer zweiten Datenbank 6 des Unterstützungssystems 1 abgelegt. Mit diesen Prädiktionsinformationen ist die Modellierung möglich, wobei diese bevorzugt ROC-Kuren enthalten. Die Prädiktionsinformationen können anhand von Studien und/oder sonstigen wissenschaftlichen Messungen bekannt sein und in die zweite Datenbank 6 eingespeichert werden; es ist jedoch auch möglich, für Untersuchungsvorgänge Prädiktionsinformationen anhand der Patientendaten der ersten Datenbank 5 und der bereits diskutierten Grundwahrheiten zu bestimmen. Einmal ermittelte aktuelle Prävalenzwerte werden als Patientendaten ebenso in die entsprechenden elektronischen Patientenakten eingespeichert und können bei Bedarf zur Anzeige gebracht werden, dienen aber grundsätzlich zur Ermittlung von Bewertungsinformationen, wie im Folgenden erläutert werden wird. Dabei können die in der ersten Datenbank 5 abgespeicherten Prävalenzwerte ständig aktuell gehalten werden, beispielsweise aktualisiert werden können, sobald Informationen zu einer neuen Patienteneigenschaft, beispielsweise einer Raucher-Anamnese, vorliegen, ein neuer Untersuchungsvorgang durchgeführt und eingetragen wurde und/oder ein sonstiges Triggerereignis eintritt, beispielsweise der Patient aufgrund seines Geburtsdatums eine neue Altersklasse eintritt.

Einer Bewertungsinformationsermittlungseinheit 11 wird dann eine Bewertungsinformation für wenigstens einen hinsichtlich der zu stellenden Diagnose durchführbaren, zukünftigen Untersuchungsvorgang ermittelt. Dabei werden sowohl die Prävalenzwerte als auch die Prädiktionsinformationen für den wenigstens einen Untersuchungsvorgang aus der zweiten Datenbank 6 berücksichtigt. Je nachdem, welche Funktionalität des Unterstützungssystems 1 gerade gewünscht ist, sind verschiedene Wege denkbar, die Bewertungsinformation zu ermitteln bzw. ihre Inhalte zu wählen, worauf bezüglich des Verfahrens hinsichtlich der Fig. 2 noch genauer eingegangen werden wird. Das Unterstützungssystem 1 umfasst ferner eine Ausgabeeinheit 12, die wenigstens teilweise auch durch Schnittstellenmittel 2 realisiert sein kann und der Ausgabe der ermittelten Bewertungsinformationen an medizinisches Personal dient. Hierzu können geeignete Anzeigevorrichtungen, beispielsweise Displays und/oder Monitore, entsprechend angesteuert werden.

Es sei schließlich noch angemerkt, dass bei der Ermittlung von Bewertungsinformationen auch externe Informationen, beispielsweise solche eines Informationssystems 13, das mit dem Unterstützungssystem 1 kommuniziert, berücksichtigt werden können. Bei dem Informationssystem 13 kann es sich beispielsweise um ein Krankenhausinformationssystem und/oder ein Radiologieinformationssystem handeln.

Fig. 2 erläutert die von dem Unterstützungssystem 1 bereitgestellten Funktionalitäten anhand eines Ausführungsbeispiels des erfindungsgemäßen Verfahrens genauer. Darin erfolgt in einem Schritt S1, wie bereits beschrieben, die Klassifizierung des Patienten in eine Personengruppe und in einem Schritt S2, wie ebenso bereits dargelegt, die Ermittlung von Prävalenzwerten.

Aufgrund von Benutzereingaben und/oder Triggerereignissen können nun verschiedene Unterstützungsfunktionen des Unterstützungssystems 1 aktiviert werden, von denen drei beispielhaft durch die Schritte S3a, S3b und S3c dargestellt sind.

Gemäß dem Schritt S3a wird ständig überprüft, ob ein aktueller Prävalenzwert bei Neuermittlung/Aktualisierung einen für eine zu stellende Diagnose spezifischen Schwellwert überschreitet, ab dem wenigstens ein bestimmter Untersuchungsvorgang/diagnostischer Test angezeigt ist. Ist der entsprechende Schwellwert überschritten, wird automatisch die Durchführung des entsprechenden Untersuchungsvorgangs empfohlen, insbesondere ergänzt um weitere Informationen zu dem zukünftigen Untersuchungsvorgang, beispielsweise prädiktiver Werte.

Der Schritt S3b betrifft eine benutzerausgelöste Auflistung zulässiger Untersuchungsvorgänge. Dürfen Untersuchungsvorgänge erst bei bestimmten Kriterien, die Prävalenzwerte auswerte, durchgeführt werden, können über diese Funktionen listenartig mögliche, also zulässige, Untersuchungsvorgänge, die die Kriterien erfüllen, angezeigt werden.

Eine besonders vorteilte Funktion wird durch den Schritt S3c beschrieben, in dem durch Modellierung und Simulation ein optimaler weiterer Diagnoseprozess ermittelt wird, mithin eine Reihenfolge durchzuführender Untersuchungsvorgänge. Die Entwicklung der Prävalenzwerte bzw. von prädiktiven Werten, hier den PPV und den NPV, wird in einer Monte-Carlo-Simulation auf einem Wahrscheinlichkeitsmodell simuliert, wobei die Modellierung auf Markov-Ketten und/oder Bayesschen Netzen beruhen kann. Die Optimierung kann sich dabei auf eine größtmögliche Sicherheit der Diagnosestellung, entweder bezüglich der positiven Diagnose (maximaler PPV) oder der negativen Diagnose (maximaler NPV) beziehen, aber auch gesundheitsökonomische Faktoren in der Optimierung berücksichtigen bzw. auf diese fokussieren, wenn hinreichend zuverlässige Diagnosen über Randbedingungen abgesichert werden. Gesundheitsökonomische Gesichtspunkte können die Schnelligkeit der Durchführung der Untersuchungsvorgänge und/oder die Kosten der Durchführung der Untersuchungsvorgänge betreffen. Bei einer Optimierung hinsichtlich mehrerer Optimierungsziele, insbesondere einer Zielfunktion, können geeignete Gewichtungsfaktoren eingesetzt werden, die insbesondere auch benutzerseitig einstellbar sein können.

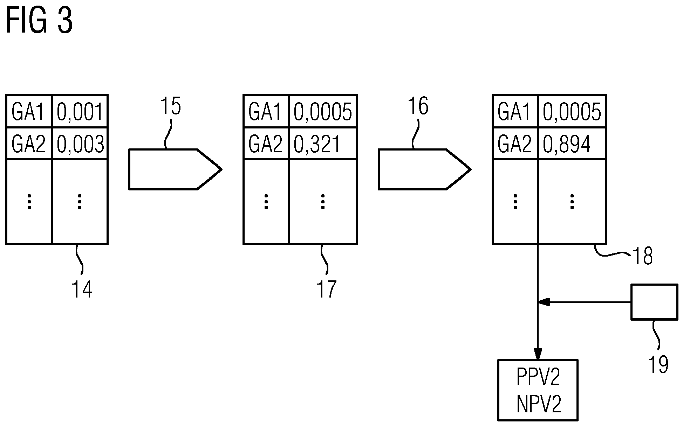

Fig. 3 illustriert die im Rahmen der vorliegenden Erfindung benutzte Evolution von Prävalenzwerten aufgrund von Untersuchungsvorgängen sowie die Prädiktion der Wirkung weiterer Untersuchungsvorgänge. In einem ersten Wahrscheinlichkeitszustand 14 liegen für mehrere Gesundheitsanomalien GA1, GA2, ... Prävalenzwerte vor, die hier noch Grundwerte allein aufgrund der Patienteneigenschaften, welche bereits eine Anamnese umfassen können, darstellen, nachdem bezüglich der zu stellenden Diagnose, die sich beispielsweise auf die Gesundheitsanomalie GA2 beziehen kann, keine Untersuchungsvorgänge vorgenommen wurden. Die Pfeile 15 symbolisieren nun die Durchführung von auf die zu stellende Diagnose bezogenen Untersuchungsvorgängen, also diagnostischen Tests, sowie die entsprechende Ergänzung der Patientendaten. Im Wahrscheinlichkeitszustand 17 haben sich ersichtlich bereits die Wahrscheinlichkeiten verändert, wobei anzumerken ist, dass Untersuchungsergebnisse für einen Untersuchungsvorgang hinsichtlich einer zu stellenden Diagnose auch Einfluss auf andere Gesundheitsanomalien und deren Prävalenzen haben können, falls sich nicht die zu stellende Diagnose auch auf mehr als eine Gesundheitsanomalie bezieht (Differentialdiagnose). Die Wahrscheinlichkeit für das Vorliegen der Gesundheitsanomalie GA2 ist ersichtlich bereits gestiegen.

Nach einem weiteren Untersuchungsvorgang, Pfeil 16, liegt ein dritter Wahrscheinlichkeitszustand 18 vor, bei dem die Wahrscheinlichkeit für das Vorliegen der Gesundheitsanomalie 2 bereits deutlich erhöht ist. Nichtsdestotrotz soll nun beispielsweise überlegt werden, welcher wenigstens eine weitere Untersuchungsvorgang durchzuführen wäre, um die Diagnose sicherstellen zu können. Hierzu werden im in Fig. 3 gezeigten Beispiel die Prädiktionsinformationen 19 der zweiten Datenbank 6 genutzt, um prädiktive Werte, also PPV und NPV, für die zu stellende Diagnose für unterschiedliche mögliche Untersuchungsvorgänge zu bestimmen, und den optimal durchzuführenden Untersuchungsvorgang auszuwählen, beispielsweise den, der den besten PPV oder NPV für die Gesundheitsanomalie 2 verspricht.

Dies sei anhand einiger Beispiele nochmals genauer erläutert. Aus Computertomographiedaten lässt sich über eine Strömungssimulation die FFR (fractional flow reserve) bestimmen, die definiert ist als der Quotient aus dem Druck nach einer Stenose und dem Druck vor der Stenose. Dabei wird normalerweise ein Wert, der kleiner als 0,75 ist, als pathologisch betrachtet. Für diesen Untersuchungsvorgang ergibt sich eine Sensitivität von ca. 90 %, eine Spezifität von ca. 70 %. Bei einem Grundwert für die Prävalenz von 1 % (allgemeine Population) ergibt sich ein positiver prädiktiver Wert des Tests von 2,9 %, das bedeutet, 2,9 % der positiv Getesteten sind tatsächlich krank. Dies ist zu wenig, um eine invasive Maßnahme zu rechtfertigen. Das Unterstützungssystem könnte daher als Bewertungsinformation beispielsweise ausgeben: "Erkrankungswahrscheinlichkeit 2,9 %, invasive Maßnahmen sind nicht gerechtfertigt". Ein niedrigerer Testwert, beispielsweise von 0,5, der eine höhere Spezifität, beispielsweise 99 %, auf Kosten der Sensitivität bedingt, erbrächte aber einen PPV, der weitere diagnostische Tests, insbesondere auch invasive Untersuchungsvorgänge, rechtfertigt (48%). Die Prädiktionsinformation in einem solchen Fall würde also das Untersuchungsergebnis (FFR) Sensitivitäten und Spezifitäten zuordnende ROC-Kurven enthalten. Für den Patienten im Beispiel würde mithin bei einem Untersuchungsergebnis von 0,5 die Bewertungsinformation eine invasive Maßnahme zur weiteren Untersuchung rechtfertigen.

Werden im genannten Beispiel weitere Voruntersuchungen berücksichtigt, beispielsweise ein hoher Kalziumwert, und ist aufgrund dieser klar, dass der Prävalenzwert für diesen Patienten deutlich höher, beispielsweise bei 30 %, liegt, ergäbe sich bereits für einen FFR von 0,75 ein PPV von 56 %, der invasive Maßnahmen rechtfertigen würde.

Das Unterstützungssystem 1 ermöglicht es, wie dargelegt wurde, auch, abzuleiten, welche weiteren diagnostischen Tests, also Untersuchungsvorgänge, mit dem geringsten Aufwand den maximalen PPV ergeben würden, wobei im genannten Beispiel weitere Risikomaße als Patientendaten einfach abgefragt werden können (Alter, Körpergewicht, Cholesterinspiegel, Blutdruck), um den Patienten der korrekten Personengruppe zuzuordnen und den Prävalenzwert genauer zu bestimmen, so dass der erwartete PPV auf einen angemessenen Wert gehoben wird oder auf einen Wert gesenkt wird, der weitere Untersuchungsvorgänge unnötig macht. Es sei darauf hingewiesen, dass das obige Beispiel selbstverständlich vereinfacht ist, nachdem in der Realität das Unterstützungssystem nicht nur den FFR und das Kalzium-Score einbeziehen würde, sondern alle auf die zu stellende Diagnose bezogenen, in der elektronischen Patientenakte enthaltenen Patientendaten.

In einem weiteren Beispiel könnte eine Empfehlung als Bewertungsinformation bezüglich eines Lymphknotens beispielsweise lauten: "Für diesen Lymphknoten sollte eine Biopsie durchgeführt werden, nachdem sie angemessen ist. Ein positives Ergebnis hätte einen prädiktiven Wert von 98 %, ein negatives von 56 %." Es sei angemerkt, dass selbstverständlich auch Prävalenzwerte, insbesondere auf Anforderung, mit ausgegeben werden können, obwohl diese selbst keine Diagnose darstellen.

Obwohl die Erfindung im Detail durch das bevorzugte Ausführungsbeispiel näher illustriert und beschrieben wurde, so ist die Erfindung nicht durch die offenbarten Beispiele eingeschränkt und andere Variationen können vom Fachmann hieraus abgeleitet werden, ohne den Schutzumfang der Erfindung zu verlassen.

**Patentansprüche**

1. Verfahren zur Unterstützung von medizinischem Personal bei der Untersuchung eines Patienten mittels wenigstens eines insbesondere eine Messung umfassenden Untersuchungsvorgangs, **dadurch gekennzeichnet, dass** unter Berücksichtigung von in einer ersten Datenbank (5) vorliegenden Patientendaten einer elektronischen Patientenakte, die wenigstens eine Eigenschaft des Patienten und/oder wenigstens ein Untersuchungsergebnis wenigstens eines bereits abgeschlossenen Untersuchungsvorgangs umfassen, wenigstens ein jeweils auf eine zu stellende Diagnose bezogener Prävalenzwert für eine den Patienten umfassende Personengruppe, zu der gehörig der Patient in Abhängigkeit wenigstens eines Teils der Patientendaten klassifiziert wurde, ermittelt wird und aus dem Prävalenzwert wenigstens eine auf wenigstens einen hinsichtlich der zu stellenden Diagnose durchführbaren, zukünftigen Untersuchungsvorgang bezogene Bewertungsinformation unter Berücksichtigung von in einer zweiten Datenbank (6) vorliegenden, die Sensitivität und/oder die Spezifität für den wenigstens einen zukünftigen Untersuchungsvorgang hinsichtlich der zu stellenden Diagnose beschreibenden Prädiktionsinformationen (19) erzeugt und an das medizinische Personal ausgegeben wird.

2. Verfahren nach Anspruch 1, **dadurch gekennzeichnet, dass** Prädiktionsinformationen (19) in Form eines Sensitivitätswertes und eines Spezifitätswertes und/oder einer ROC-Kurve verwendet werden.

3. Verfahren nach Anspruch 1 oder 2, **dadurch gekennzeichnet, dass** der Prävalenzwert wenigstens teilweise ausgehend von einem Grundwert für eine durch Patienteneigenschaften definierte Personengruppe unter Berücksichtigung vorliegender Untersuchungsergebnisse und der dem bereits abgeschlossenen Untersuchungsvorgang zugeordneten Prädiktionsinformation (19) bestimmt wird.

4. Verfahren nach einem der vorangehenden Ansprüche, **dadurch gekennzeichnet, dass** der Grundwert und/oder Prävalenzwert für die jeweilige Personengruppe und/oder die Prädiktionsinformation (19) für einen Untersuchungsvorgang wenigstens teilweise in Abhängigkeit von Patientendaten von weiteren in der ersten Datenbank (5) erfassten, der jeweiligen Personengruppe zugehörigen bzw. dem Untersuchungsvorgang unterzogenen Patienten, zu denen eine Grundwahrheit bezüglich der zu stellenden Diagnose bekannt oder ableitbar ist, ermittelt wird.

5. Verfahren nach einem der vorangehenden Ansprüche, **dadurch gekennzeichnet, dass** eine Bewertungsinformation für wenigstens einen zukünftigen Untersuchungsvorgang automatisch erzeugt wird, wenn ein Prävalenzwert einen insbesondere für die zu stellende Diagnose spezifischen Schwellwert überschreitet.

6. Verfahren nach einem der vorangehenden Ansprüche, **dadurch gekennzeichnet, dass** die Bewertungsinformation wenigstens einen positiven prädiktiven Wert und/oder wenigstens einen negativen prädiktiven Wert umfassend ermittelt wird, insbesondere für einen benutzerseitig ausgewählten Untersuchungsvorgang.

7. Verfahren nach einem der vorangehenden Ansprüche, **dadurch gekennzeichnet, dass** die Bewertungsinformation wenigstens einen empfohlenen Untersuchungsvorgang, insbesondere eine Reihe empfohlener Untersuchungsvorgänge, umfassend ermittelt wird.

8. Verfahren nach Anspruch 7, **dadurch gekennzeichnet, dass** als Bewertungsinformation eine wenigstens einen Untersuchungsvorgang umfassende Reihenfolge von Untersuchungsvorgängen in einem Optimierungsverfahren ermittelt wird, wobei die Optimierung hinsichtlich einer Zuverlässigkeit einer positiven Diagnose und/oder einer Zuverlässigkeit einer negativen Diagnose und/oder der Kostengünstigkeit und/oder des Zeitbedarfs für die Untersuchungsvorgänge erfolgt und/oder wenigstens eine auf eine minimale Zuverlässigkeit abzielende Randbedingung verwendet wird.

9. Verfahren nach einem der vorangehenden Ansprüche, **dadurch gekennzeichnet, dass** zur Ermittlung der Bewertungsinformation der wenigstens eine zukünftige Untersuchungsvorgang in einem mathematischen Wahrscheinlichkeitsmodell berechnet und/oder simuliert wird.

10. Verfahren nach einem der vorangehenden Ansprüche, **dadurch gekennzeichnet, dass** die Bewertungsinformation eine Auflistung von Untersuchungsvorgängen umfasst, für die der Prävalenzwert einen deren Zulässigkeit beschreibenden Grenzwert überschreitet.

11. Verfahren nach einem der vorangehenden Ansprüche, **dadurch gekennzeichnet, dass** bei der Ermittlung der Bewertungsinformation wenigstens eine Verfügbarkeitsinformation bezüglich des wenigstens einen zukünftigen Untersuchungsvorgangs, insbesondere aus einem Informationssystem (13) abgerufen, berücksichtigt wird.

12. Verfahren nach einem der vorangehenden Ansprüche, **dadurch gekennzeichnet, dass** bei einem mittels eines medizintechnischen Untersuchungsgeräts (3) durchzuführenden zukünftigen Untersuchungsvorgang ein auf die Personengruppe abgestimmtes Untersuchungsprotokoll als Bewertungsinformation ermittelt und an das Untersuchungsgerät (3) übermittelt und/oder dort ausgeführt wird und/oder wenigstens die Anzeige der Bewertungsinformation an dem Untersuchungsgerät (3) erfolgt.

13. Unterstützungssystem (1), aufweisend

- eine erste Datenbank (5) mit elektronischen Patientenakten mehrerer Patienten, die jeweils Patientendaten mit wenigstens einer Eigenschaft des Patienten und/oder wenigstens einem Untersuchungsergebnis wenigstens eines bereits abgeschlossenen Untersuchungsvorgangs an dem Patienten enthalten,
- eine zweite Datenbank (6) mit die Sensitivität und/oder die Spezifität für jeweils einen Untersuchungsvorgang hinsichtlich jeweils einer zu stellenden Diagnose beschreibenden Prädiktionsinformationen (19),
- eine Klassifizierungseinheit (8) zur Zuordnung eines Patienten zu einer Personengruppe in Abhängigkeit wenigstens eines Teils der Patientendaten des Patienten und einer zu stellenden Diagnose,
- eine Prävalenzermittlungseinheit (9) zur Ermittlung eines auf die zu stellende Diagnose bezogenen Prävalenzwerts für den Patienten in Abhängigkeit der Personengruppe und/oder der Patientendaten,
- eine Bewertungsinformationsermittlungseinheit (11) zur Ermittlung einer auf wenigstens einen hinsichtlich der zu stellenden Diagnose durchführbaren, zukünftigen Untersuchungsvorgang bezogenen Bewertungsinformation unter Berücksichtigung des Prävalenzwertes für die zu stellende Diagnose und den Patienten und der Prädiktionsinformationen (19) für den wenigstens einen Untersuchungsvorgang, und
- eine Ausgabeeinheit (12) für die Bewertungsinformation.

14. Computerprogramm, welches die Schritte eines Verfahrens nach einem der Ansprüche 1 bis 12 durchführt, wenn es auf einer Recheneinrichtung (7) ausgeführt wird.

15. Elektronisch lesbarer Datenträger, auf dem ein Computerprogramm nach einem der vorangehenden Ansprüche gespeichert ist.

FIG 1

5

6

10

1

8    9    11    12    13

7

2

3

2

4

FIG 2

S1

S2

S3a    S3b    S3c

FIG 3

| | |
|-----|-------|
| GA1 | 0,001 |
| GA2 | 0,003 |
| ⋮ | ⋮ |

15

| | |
|-----|--------|
| GA1 | 0,0005 |
| GA2 | 0,321 |
| ⋮ | ⋮ |

16

| | |
|-----|--------|
| GA1 | 0,0005 |
| GA2 | 0,894 |
| ⋮ | ⋮ |

18

14

17

19

PPV2
NPV2

Europäisches
Patentamt

European
Patent Office

Office européen
des brevets

**EUROPÄISCHER RECHERCHENBERICHT**

Nummer der Anmeldung

EP 17 16 9049

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | WO 2016/097886 A1 (L I A DI GIUSEPPE CAPASSO [IT]) 23. Juni 2016 (2016-06-23) * Das ganze Dokument, insbesondere: Anspruch 1, Seiten 4, 8, 9, 11, 12, 14, 15, 17-19, 23, 24, 31 - 35. * | 1-15 | INV. G06F19/00 |
| A | KAVISHWAR B WAGHOLIKAR ET AL: "Modeling Paradigms for Medical Diagnostic Decision Support: A Survey and Future Directions", JOURNAL OF MEDICAL SYSTEMS, KLUWER ACADEMIC PUBLISHERS-PLENUM PUBLISHERS, NE, Bd. 36, Nr. 5, 1. Oktober 2011 (2011-10-01), Seiten 3029-3049, XP035103437, ISSN: 1573-689X, DOI: 10.1007/S10916-011-9780-4 * Das ganze Dokument, insbesondere: Kapitel Introduction und Results. * | 1-11, 13-15 | |
| A | WO 2008/031082 A2 (PORWANCHER RICHARD [US]) 13. März 2008 (2008-03-13) * Das ganze Dokument, insbesondere: Anspruch 1, Absatz [0022] - Absatz [0034]. * | 1-9, 13-15 | RECHERCHIERTE SACHGEBIETE (IPC) G06F |
| A,D | US 8 401 872 B2 (HAIDER SULTAN [DE] ET AL) 19. März 2013 (2013-03-19) * Absatz [0004] - Absatz [0005]; Ansprüche 1,4,7 * | 8,12 | |
| A,D | US 7 593 913 B2 (WANG LU-YONG [US] ET AL) 22. September 2009 (2009-09-22) * Das ganze Dokument, insbesondere: Ansprüche 1 und 7, Spalten 1, 2, 5 - 8. * | 1-7, 13-15 | |

Der vorliegende Recherchenbericht wurde für alle Patentansprüche erstellt

| Recherchenort | Abschlußdatum der Recherche | Prüfer |
|---|---|---|
| München | 16. Juni 2017 | Nagele, Stefan |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

**ANHANG ZUM EUROPÄISCHEN RECHERCHENBERICHT**
**ÜBER DIE EUROPÄISCHE PATENTANMELDUNG NR.**

EP 17 16 9049

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

16-06-2017

| Im Recherchenbericht angeführtes Patentdokument | Datum der Veröffentlichung | Mitglied(er) der Patentfamilie | Datum der Veröffentlichung |
|---|---|---|---|
| WO 2016097886 A1 | 23-06-2016 | KEINE | |
| WO 2008031082 A2 | 13-03-2008 | EP 2084535 A2 | 05-08-2009 |
| | | PL 2084535 T3 | 30-12-2016 |
| | | SI 2084535 T1 | 31-08-2016 |
| | | US 2008064118 A1 | 13-03-2008 |
| | | US 2011295516 A1 | 01-12-2011 |
| | | WO 2008031082 A2 | 13-03-2008 |
| US 8401872 B2 | 19-03-2013 | CN 1969774 A | 30-05-2007 |
| | | DE 102005055657 A1 | 24-05-2007 |
| | | US 2007161871 A1 | 12-07-2007 |
| US 7593913 B2 | 22-09-2009 | DE 102007001406 A1 | 09-08-2007 |
| | | US 2007168308 A1 | 19-07-2007 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 7593913 B2
- US 8238999 B2
- US 8321238 B2
- US 8401872 B2
- US 8640053 B2